# EUROPÄISCHE PATENTSCHRIFT

(11) **EP 3 538 890 B1**
(45) Veröffentlichungstag und Bekanntmachung des Hinweises auf die Patenterteilung: **21.06.2023**
(21) Anmeldenummer: 17808348.1
(22) Anmeldetag: 09.11.2017
(51) Int. Cl.: G01N 33/543, G01N 33/569, G01N 33/58

(54) **ULTRASENSITIVER NACHWEIS VON VIRUS- UND VIRUS-ÄHNLICHEN PARTIKELN**
ULTRASENSITIVE DETECTION OF VIRUS PARTICLES AND VIRUS-LIKE PARTICLES
DÉTECTION ULTRASENSIBLE DE PARTICULES VIRALES ET DE PARTICULES ANALOGUES AUX PARTICULES VIRALES

(30) Priorität: 09.11.2016 DE 102016121455
(43) Veröffentlichungstag der Anmeldung: 18.09.2019
(73) Patentinhaber: Forschungszentrum Jülich GmbH, 52425 Jülich (DE)
(72) Erfinder: WILLBOLD, Dieter, 52425 Jülich (DE); BANNACH, Oliver, 40595 Düsseldorf (DE); KULAWIK, Andreas, 40699 Erkrath (DE); ZAFIU, Christian, A - 1030 Wien (AT)
(74) Vertreter: Fitzner, Uwe
(86) Internationale Anmeldenummer: PCT/EP2017/078777
(87) Internationale Veröffentlichungsnummer: WO 2018/087236

(56) Entgegenhaltungen:
- WO-A1-99/15900
- WO-A1-2011/130332
- WO-A1-2014/110127
- WO-A2-2010/017381
- US-A1- 2002 081 569
- US-A1- 2012 028 246
- US-A1- 2015 175 975
- J R Crowther ET AL: "Q uan .tification of whole virus particles (146s) of foot-and-mouth disease virus in the presence of virus subunits (12S), using monoclonal antibodies in a sandwich ELISA 0264-410X(95)00051-8", Vaccine, 1. Januar 1995 (1995-01-01), Seiten 1064-75, XP055445386, Gefunden im Internet: URL:https://ac.els-cdn.com/0264410X9500051 2/1-s2.0-0264410X95000512-main.pdf?_tid=32 17eb9e-02bd-11e8-b06e-00000aab0f26&acdnat= 1516987405_e99c8f28300024f91aadc665a89ec75 a
- JANISSEN R ET AL: "Optimized straight forward procedure for covalent surface immobilization of different biomolecules for single molecule applications", COLLOIDS AND SURFACES. B, BIOINTERFACES, ELSEVIER, AMSTERDAM, NL, Bd. 71, Nr. 2, 1. Juli 2009 (2009-07-01), Seiten 200-207, XP026099855, ISSN: 0927-7765, DOI: 10.1016/J.COLSURFB.2009.02.011 [gefunden am 2009-02-21] in der Anmeldung erwähnt
- EBNER ET AL: "Comparison of different aminofunctionalization strategies for attachment of single antibodies to AFM cantilevers", ULTRAMICROSCOPY, ELSEVIER, AMSTERDAM, NL, Bd. 107, Nr. 10-11, 27. Juli 2007 (2007-07-27), Seiten 922-927, XP022174224, ISSN: 0304-3991, DOI: 10.1016/J.ULTRAMIC.2007.02.035
- J. B. Munro ET AL: "Conformational dynamics of single HIV-1 envelope trimers on the surface of native virions", Science, vol. 346, no. 6210, 8 October 2014 (2014-10-08), pages 759-763, XP55731342, ISSN: 0036-8075, DOI: 10.1126/science.1254426

## Beschreibung

Die vorliegende Erfindung betrifft ein Verfahren zur quantitativen und/oder qualitativen Bestimmung von Viruspartikeln enthaltend mindestens eine Bindungsstelle für ein Fängermolekül und mindestens eine Bindungsstelle für eine Sonde, ein Kit zu dessen Durchführung sowie verschiedene Anwendungen.

Viren werden als infektiöse Partikel definiert, die sich nur innerhalb geeigneter Wirtszellen vermehren können. Viren sind aufgebaut aus Nukleinsäure (DNA oder RNA), Proteinen und teilweise auch Lipiden. Dazu gehören auch Bakteriophagen, die Bakterien infizieren. Da sie alle weder eine eigenständige Replikation noch einen eigenen Stoffwechsel besitzen, sind sie nach herrschender Meinung nicht den Lebewesen zuzurechnen. Die einzelnen Viruspartikel bestehen aus einem Genom, der oben erwähnten Nukleinsäure und einer Proteinhülle. Manche besitzen eine zusätzliche Hülle. Ein solches, komplettes, infektiöses Viruspartikel stellt die extrazelluläre Form des Virus dar und wird auch als Virion bezeichnet. Aufgrund der geringen Größe der infektiösen Viruspartikel, je nach Spezies zwischen 15 nm und 300 nm, sind bisher nur wenige direkte Nachweismethoden bekannt; die meisten Nachweise beruhen auf den Symptomen der infizierten Zellen oder Lebewesen. Als Infektion wird allgemein das aktive oder passive Eindringen, Verbleiben und anschließendes Vermehren von Pathogenen, wie z.B. Viruspartikel, in einem Organismus bezeichnet. Sofern die Wirtszellen den Prokaryonten zuzuordnen sind, werden die infektiösen Viruspartikel Bakteriophagen genannt.

Als bisher einziger, direkter Nachweis von Viruspartikeln wird aufgrund der geringen Größe der Partikel die Elektronenmikroskopie eingesetzt. Dabei handelt es sich um einen Nachweis von Viren auf Basis ihrer Eigenschaften als Partikel. Neben den hohen Kosten für die entsprechende Ausrüstung ermöglicht die Elektronenmikroskopie lediglich eine Unterscheidung zwischen verschiedenen Virusfamilien, aber keine Bestimmung entsprechender Subspezies. Des Weiteren müssen die Proben chemisch oder physikalisch fixiert werden, so dass keine funktionsfähigen Proteine mehr vorliegen beziehungsweise Epitope denaturiert oder maskiert werden. Außerdem ist das Vorliegen einer hohen Anzahl an Viruspartikeln notwendig.

Nach aktuellem Stand der Technik erfolgt der Nachweis von Viren bzw. viraler Bestandteile mittels funktionaler, antikörperbasierter oder genombasierter Techniken, wie z.B. PCR-basierte Vervielfältigung von viraler RNA oder DNA und Identifikation mittels Hybridisierungsabängiger Sonden.

Der Nachweis funktionaler Viren erfolgt unter Ausnutzung des cytopathischen Effekts mittels Plaque Assay. Inaktivierte Viren oder nicht-infektiöse Partikel werden nicht erfasst. Genombasierte Techniken quantifizieren eher die Menge an viraler RNA/DNA als die Menge an Virus-Partikeln.

Kontinuierliche Epitope viraler Proteine können im Western Blot mit Antikörpern nachgewiesen werden.

Bei dringendem Verdacht auf eine virale Infektion und wenn andere Nachweisverfahren kein positives Ergebnis liefern wird die PCR-Technik eingesetzt. Dabei wird das Genom unter Einsatz Virus-spezifischer Primer mittels Polymerasekettenreaktrion amplifiziert. Bei RNA-Viren ist die Umschreibung von RNA in DNA mittels reverser Transkriptase dafür Voraussetzung. Der Nachweis des Amplifikats erfolgt durch Hybridisierung durch spezifische Sonden oder durch unspezifische Färbung der amplifizierten DNA und Längen-abhängige Identifikation nach Gelelektrophorese.

PCR-basierte Methoden müssen aufwendig kalibriert werden, sind nicht strikt quantitativ und sehr anfällig für Kontaminationen, die zu einem falsch-positivem Ergebnis führen können. Sie bestimmen eigentlich die Anwesenheit von viraler DNA/RNA oder manchmal sogar nur Teilen davon. Außerdem können Probenkomponenten, welche die PCR-Reaktion inhibieren, ein falsch-negativen Ergebnis verursachen. Daher müssen die Proben stets gereinigt werden, bevor sie analysiert werden können.

Immuntests, welche körpereigene, antivirale Antikörper nachweisen, können i.d.R. erst Wochen nach Infektion zu einem positiven Ergebnis führen (diagnostische Lücke). ELISA-Verfahren weisen in der Regel nur die Anwesenheit von Teilfragmenten eines Virus nach und nicht intakte Partikel.

B. Munro ET AL: "Conformational dynamics of single HIV-1 envelope trimers on the surface of native virions", Science, Bd. 346, Nr. 6210, 8. Oktober 2014, Seiten 759-763 beschäftigt sich mit der Analyse von Konformationsdynamiken in HIV-1-Virionen.

US 2012/028246 A1 (NAKAYA TAKAAKI [JP] ET AL) 2. Februar 2012 enthüllt Verfahren zur quantitativen und/oder qualitativen Bestimmung von Viruspartikeln.

Aufgabe der vorliegenden Erfindung ist es einen ultrasensitiven Nachweis für Viruspartikel zur Verfügung zu stellen. Weitere Aufgabe ist es, ein Verfahren zur Verfügung zu stellen, mit welchem der Nachweis von Virus- und Virus-ähnlichen Partikeln in beliebigen Proben und in möglichst geringer Anzahl, mithin auch ein Einzelnachweis in beliebigen Proben möglich ist, insbesondere auch für nicht-kultivierbare Viren. Dadurch ist ein Nachweis viraler Infektionen und auch Kontaminationen in beliebigen Proben durchführbar.

Ferner soll nicht nur ein qualitativer Nachweis von Viruspartikeln möglich sein, sondern auch eine Quantifizierung und Charakterisierung von Viruspartikeln in beliebigen Proben. Dadurch soll einerseits eine direkte und absolute Quantifizierung der Partikelzahl und andererseits eine genaue Charakterisierung der Viruspartikel gewährleistet sein und so eine Typisierung ermöglichen. Die Ergebnisse sollen in der therapiebegleitenden Diagnostik, Differentialdiagnostik und/oder Analyse der Virus-Assemblierung verwendbar sein.

Des Weiteren soll das Verfahren auch den Nachweis von Protein-Protein-Interaktionen, also der Wirts-Virus-Interaktionen, gewährleisten.

Der Nachweis soll mit wenigen, einfachen Schritten unmittelbar aus beliebigen Proben wie z.B. ex vivo aus Körperflüssigkeiten oder Autopsie- oder Biopsie Material, Organen, aber auch Proben aus der Umwelt, wie z. B. Wasser-, Pflanzen- und Bodenproben, sowie Lebensmitteln möglich sein.

Diese Aufgaben werden gelöst durch ein Verfahren zur quantitativen und/oder qualitativen Bestimmung von Viruspartikeln enthaltend mindestens eine Bindungsstelle für ein Fängermolekül und mindestens eine Bindungsstelle für eine Sonde umfassend folgende Schritte:a) Immobilisieren von Fängermolekülen auf einem Substrat,b) In Kontakt bringen der Viruspartikel mit den Fängermolekülen, wobei die Fängermoleküle kovalent an das Substrat gebunden sind,c) Immobilisieren der Viruspartikel auf dem Substrat durch Bindung an Fängermoleküle,d) In Kontakt bringen der Viruspartikel mit den Sonden unde) Binden der Sonden an die Viruspartikel, wobei die Sonden ein Viruspartikelaffines Molekül oder Molekülteil, welches eine Bindungsstelle des Viruspartikels erkennt und daran bindet, umfassen,wobei die Sonden in der Lage sind, ein spezifisches Signal zu emittieren und die Schritte b) und d) gleichzeitig oder d) vor b) erfolgen können,wobei eine ortsaufgelöste Bestimmung des Sondensignals erfolgt, wobei die Detektion mittels ortsauflösender Fluoreszenzmikroskopie erfolgt, wobei zur Detektion TIRF eingesetzt wird.

Sofern die Schritte b) und d) gleichzeitig erfolgen, werden somit auch die Schritte c) und e) gleichzeitig durchgeführt.

In der weiteren Variante, in welcher Schritt d) vor Schritt b) durchgeführt wird, erfolgt somit in Schritt c) eine Immobilisierung von mit Sonden markierten Viruspartikeln auf dem Substrat. Mithin erfolgt also auch Schritt e) vor den Schritten b) und c).

Im Sinne der vorliegenden Erfindung bedeutet die "quantitative Bestimmung" zunächst die Bestimmung der Konzentration der Viruspartikel, mithin also auch die Bestimmung ihrer An- und oder -Abwesenheit.

Bevorzugt bedeutet die quantitative Bestimmung auch die selektive Quantifizierung bestimmter Virustypen. Eine solche Quantifizierung kann über die entsprechenden Sonden gesteuert werden.

Im Sinne der vorliegenden Erfindung bedeutet die "qualitative Bestimmung" die Charakterisierung der Viruspartikel, wie zum Beispiel die Bestimmung der Form.

Die Viruspartikel werden mit einer oder mehreren für die Detektion dienlichen Sonden markiert. In einer Variante werden mindestens zwei, drei, vier fünf, sechs, sieben oder mehr Sonden eingesetzt. In einer weiteren Variante werden zwei, drei, vier fünf, sechs, sieben oder mehr unterschiedliche Sonden eingesetzt.

Die Sonden enthalten ein Viruspartikel-affines Molekül oder Molekülteil, welches eine Bindungsstelle des Viruspartikels erkennt und daran bindet. Außerdem enthalten die Sonden mindestens ein Detektionsmolekül oder Molekülteil, welches an das Viruspartikel-affine Molekül oder Molekülteil kovalent gebunden ist und mittels chemischer oder physikalischer Verfahren detektierbar und messbar ist.

Die Sonden können in einer Alternative identische affine Moleküle oder Molekülteile mit unterschiedlichen Detektionsmolekülen (oder Teile) aufweisen. In einer weiteren Alternative können unterschiedliche affine Moleküle oder Molekülteile mit unterschiedlichen Detektionsmolekülen oder Teilen kombiniert sein, oder alternativ unterschiedliche affine Moleküle oder Teile mit identischen Detektionsmolekülen oder Teilen. Es können auch Mischungen verschiedener Sonden eingesetzt werden.

Der Einsatz mehrerer unterschiedlicher Sonden, die an unterschiedliche Detektionsmoleküle oder Molekülteile gekoppelt sind, erhöht zum einen die Spezifität des Signals (Korrelationssignals), zum anderen ermöglicht dies die Identifikation von Viruspartikeln, die sich in einem oder mehreren Merkmalen unterscheiden. Dies ermöglicht eine selektive Quantifizierung und Charakterisierung der Viruspartikel.

In einer Ausführung erfolgt eine ortsaufgelöste Bestimmung des Sondensignals, also eine ortsaufgelöste Detektion des Signals, welches von der Sonde emittiert wird. Demgemäß sind in dieser Ausführung der Erfindung Verfahren, die auf einem nicht-ortsaufgelösten Signal beruhen, wie ELISA oder Sandwich-ELISA, ausgeschlossen. Darunter fallen auch ELISA-ähnliche Verfahren, also alle Verfahren, die auf einem nicht-ortsaufgelösten Signal beruhen, sondern auf "Bulk"-Messungen, mit anderen Worten: Ensemble-Messungen; mithin alle Immun-Assays, unabhängig, ob die Detektion auf einer enzymatischen Farbreaktion beruht, oder auf Detektion von Fluroreszenz, magnetisch oder radioaktiv markierten Sonden bzw. Antikörper, sofern nicht das Signal einzelner Partikeln, sondern von ganzen Volumensegmenten detektiert wird.

Das erfindungsgemäße Verfahren ist ferner gekennzeichnet durch folgende Merkmale:
Die Bestimmung intakter, nicht zerstörter Virus-Partikel (also nicht zerstörter Viren); die Bestimmung der Anzahl dieser Partikel und/oder Form; Einzelpartikel-Untersuchung bzw. -Analyse, mithin keine Ensemble-Messung; Bestimmung und Analyse von geringen Konzentrationen von 100 Partikel/µl oder kleiner; Unterscheidung zwischen leeren Virushüllen und Viruspartikel enthaltend neben der Hülle weitere Bestandteile wie z.B. DNA, RNA, Proteine unterschiedlich jenen der Hülle;

Bei der Detektion ist eine hohe Ortsauflösung vorteilhaft aber nicht essentiell. In einer Ausführung des erfindungsgemäßen Verfahrens werden dabei so viele Datenpunkte gesammelt, dass die Detektion eines Virus oder virusähnlichen Partikel vor einem Hintergrundsignal, welches z.B. durch gerätespezifisches Rauschen, andere unspezifische Signale oder unspezifisch gebundene Sonden verursacht wird, ermöglicht. Auf diese Weise werden so viele Werte ausgelesen (Read-out-Werte), wie ortsaufgelöste Ereignisse, wie z.B. Pixel, vorhanden sind. Durch die Ortsauflösung wird jedes Ereignis vor dem jeweiligen Hintergrund bestimmt und stellt so einen Vorteil gegenüber ELISA-Verfahren ohne ortsaufgelöstem Signal dar.

In einer Ausführung beruht die ortsaufgelöste Bestimmung des Sondensignals auf der Untersuchung eines kleinen Volumenelements im Vergleich zum Volumen der Probe, im Bereich von wenigen Femtolitern bis unterhalb eines Femtoliters, oder eines Volumenbereichs überhalb der Kontaktoberfläche der Fängermoleküle mit einer Höhe von von 500 nm, bevorzugt 300 nm, besonders bevorzugt 250 nm, insbesondere 200 nm.

Im Sinne der Erfindung werden Viruspartikel ausgewählt aus der Gruppe enthaltend oder bestehend aus Virus, Virion, Bakteriophage und Teile oder Fragmente davon. Virusähnlichen Partikel sind zum Beispiel Virushüllen, Teile oder Fragmente davon die nicht replikationsfähig sind. Der Begriff Virus umfasst im Sinne der Erfindung auch virusähnliche Partikel sowie jeweils Teile oder Fragmente von Viren und/oder virusähnlichen Partikel.

Taxonomisch können Viren in Viren eingeteilt werden, die ein Kapsid, also eine Hülle aus Proteinen aufweisen, und Viren, die eine Hülle aus Lipiden, einer Lipid-Doppelmembran mit eingelagerten viralen Proteinen aufweisen. Im Sinne der Erfindung können alle erfindungsgemäßen Viruspartikel dementsprechend eingeteilt werden, mithin in Partikel, die eine Hülle oder Teile einer Hülle enthaltend Lipide aufweisen und gegebenenfalls zusätzlich eine Hülle oder Teile einer Hülle aus Proteinen, und Partikel, die lediglich eine Hülle oder Teile einer Hülle aus Proteinen aufweisen.

Aufgrund der Möglichkeit, einzelne Partikel zu detektieren und zu analysieren, können bei entsprechender Auswahl unterschiedliche Fänger und Sonden auch parallel in einer Probe unterschiedliche Viren analysiert werden. Mithin kann das Verfahren auch zur Differentialdiagnose verwendet werden.

Das Verfahren wird nicht im und/oder am menschlichen Körper durchgeführt, sondern ex vivo, mithin in vitro.

Erfindungsgemäß handelt es sich bei den Teilen oder Fragmenten der Viruspartikel um Teile die mindestens zwei Bindungsstellen enthalten.

In einer Ausführung wird das Material des Substrats ausgewählt aus der Gruppe enthaltend oder bestehend aus Kunststoff, Silizium und Siliziumdioxid. In einer bevorzugten Alternative wird als Substrat Glas eingesetzt.

Gemäß der Erfindung sind die Fängermoleküle kovalent an das Substrat gebunden.

Hierzu wird in einer Alternative ein Substrat eingesetzt, das eine hydrophile Oberfläche aufweist. In einer Alternative wird dies durch das Auftragen einer hydrophilen Schicht, vor Schritt a), auf das Substrat erreicht. Mithin binden die Fängermoleküle kovalent an das Substrat bzw. an die hydrophile Schicht, mit welchen das Substrat beladen ist.

Die hydrophile Schicht ist eine Biomolekül-abweisende Schicht, so dass die unspezifische Bindung von Biomolekülen an das Substrat minimiert wird. Auf diese Schicht werden die Fängermoleküle, bevorzugt kovalent immobilisiert. Diese sind affin gegenüber einem Merkmal der Viruspartikel. Die Fängermoleküle können alle identisch sein, oder Mischungen unterschiedlicher Fängermoleküle sein. In einer Alternative werden als Fängermoleküle und Sonden dieselben Moleküle eingesetzt, bevorzugt enthalten die Fängermoleküle kein Detektionsmolekül bzw. Molekülteile.

In einer Ausführung wird die hydrophile Schicht ausgewählt aus der Gruppe enthaltend oder bestehend aus PEG, Poly-Lysin, bevorzugt Poly-D-Lysin, und Dextran oder Derivate davon, bevorzugt Carboxymethyl-Dextran (CMD). Derivate im Sinne der Erfindung sind Verbindungen die sich in einigen Substituenten von den Stammverbindungen unterscheiden, wobei die Substituenten gegenüber dem erfindungs-gemäßen Verfahren inert sind.

In einer Ausführung wird die Oberfläche des Substrats vor Auftrag der hydrophilen Schicht zunächst hydroxyliert und anschließend mit geeigneten chemischen Gruppen, bevorzugt Aminogruppen, funktionalisiert . Diese Funktionalisierung mit Aminogruppen erfolgt in einer Alternative durch in Kontaktbringen des Substrats mit Aminosilanen, bevorzugt APTES (3-Aminopropyltrietoxysilan), oder mit Ethanolamin.

Zur Vorbereitung des Substrats auf die Beschichtung werden ein oder mehrere der folgenden Schritte durchgeführt:
- Waschen eines Substrats aus Glas bzw. eines Glasträgers im Ultraschallbad oder Plasma-cleaner, alternativ dazu in 5 M NaOH mindestens 3 Stunden inkubieren,
- Spülen mit Wasser und anschließendem Trocknen unter Stickstoff oder unter Vakuum,
- Eintauchen in eine Lösung aus konzentrierter Schwefelsäure und Wasserstoffperoxid im Verhältnis 3:1 für die Aktivierung der Hydroxylgruppen,
- Spülen mit Wasser bis zu einer neutralen pH, anschließend mit Ethanol und Trocknen unter Stickstoffatmosphäre,
- Eintauchen in eine Lösung mit 3-Aminopropyltrietoxysilan (APTES) (1 - 7 %), bevorzugt in trockenem Toluol, oder einer Lösung von Ethanolamin,
- Spülen mit Aceton oder DMSO und Wasser und Trocknen unter Stickstoffatmosphäre.

In einer Alternative erfolgt das in Kontakt bringen des Substrats mit Aminosilanen, bevorzugt APTES, in der Gasphase; das gegebenenfalls vorbehandelte Substrat wird mithin mit den Aminosilanen bedampft.

Für die Beschichtung mit Dextran, bevorzugt Carboxymethyl-Dextran (CMD), wird das Substrat mit einer wässrigen Lösung von CMD (in einer Konzentration von 10 mg/ml oder 20 mg/ml) und gegebenenfalls N-Ethyl-N-(3-Dimethylaminpropyl) Carbodiimid (EDC), (200 mM) und N-Hydroxysuccinimid (NHS), (50 mM) inkubiert und anschließend gewaschen.

Das Carboxymethyl-Dextran ist in einer Variante kovalent an die Glasoberfläche gebunden, die zunächst hydroxyliert und anschließend mit Amingruppen funktionalisiert wurde, wie oben beschrieben.

Als Substrat können auch Mikrotiterplatten, bevorzugt mit Glasboden, eingesetzt werden. Da bei der Verwendung von Polystryrolrahmen der Einsatz von konzentrierter Schwefelsäure nicht möglich ist, erfolgt die Aktivierung der Glasoberfläche in einer Ausführungsvariante der Erfindung analog Janissen et al. (Colloids Surf B Biointerfaces, 2009, 71 (2), 200-207).

Auf diese hydrophile Schicht werden (bevorzugt kovalent) Fängermoleküle immobilisiert, welche affin gegenüber einem Merkmal (z.B.: Proteine) des zu detektierenden virusähnlichen oder Viruspartikel sind. Die Fängermoleküle können alle identisch oder Mischungen verschiedener Fängermoleküle sein.

In einer Ausführung der vorliegenden Erfindung werden die Fängermoleküle (bevorzugt Antikörper), gegebenenfalls nach einer Aktivierung des mit CMD beschichteten Trägers durch eine Mischung aus EDC/NHS (200 bzw. 50 mM), auf dem Substrat immobilisiert. Verbleibende Carboxylat-Endgruppen, an die keine Fängermoleküle gebunden wurden, können deaktiviert werden. Zur Deaktivierung dieser Carboxylat-Endgruppen auf dem CMD-Spacer wird Ethanolamin in DMSO verwendet. Vor dem Auftrag der Proben werden die Substrate bzw. Träger gegebenenfalls mit PBS gespült.

Die zu vermessende Probe wird mit dem so präparierten Substrat in Kontakt gebracht und gegebenenfalls inkubiert. Als zu untersuchende Probe können körpereigene Flüssigkeiten oder Gewebe eingesetzt werden. In einer Ausführung der vorliegenden Erfindung wird die Probe ausgewählt aus Liquor (CSF), Blut, Plasma und Urin. Aber auch Lebensmittel und Abstriche von Gegenständen dienen als Proben. Die Proben können unterschiedliche, dem Fachmann bekannte, Aufbereitungsschritte durchlaufen.

In einer Ausführung der vorliegenden Erfindung erfolgt das Auftragen der Probe unmittelbar auf dem Substrat (nichtbeschichteten Substrat), gegebenenfalls durch kovalente Bindung auf der gegebenenfalls aktivierten Oberfläche des Substrats.

In einer Variante der vorliegenden Erfindung erfolgt eine Vorbehandlung der Probe nach einem oder mehreren der folgenden Verfahren:
- Erhitzen der Probe,
- Ein oder mehrere Gefrierauftauzyklen,
- Mechanischer Aufschluss,
- Homogenisierung der Probe,
- Verdünnen mit Wasser oder Puffer,
- Behandlung mit Enzymen, zum Beispiel Proteasen, Nuklease, Lipasen,
- Zentrifugieren,
- Präzipitation,
- Kompetition mit Sonden, um eventuell vorhandene Antikörper zu verdrängen.

Bevorzugt wird die Probe unmittelbar und/oder ohne Vorbehandlung mit dem Substrat in Kontakt gebracht.

Unspezifisch gebundene Substanzen können durch Waschschritte entfernt werden.

In einem weiteren Schritt werden immobilisierte virusähnliche Partikel oder Viruspartikel mit einer oder mehreren für die weitere Detektion dienlichen Sonden markiert. Wie oben beschrieben können die einzelnen Schritte auch in einer anderen Reihenfolge erfindungsgemäß durchgeführt werden.

Durch geeignete Waschschritte werden überschüssige Sonden, die nicht an die Viruspartikel gebunden sind, entfernt.

In einer Alternative werden diese überschüssigen Sonden nicht entfernt. Dadurch entfallen die letzten Waschschritte und es findet auch keine Gleichgewichtsverschiebung in Richtung der Dissoziation der Viruspartikel-Sonden-Komplexe oder -Verbindungen statt. Durch die orts-aufgelöste Detektion werden die überschüssigen Sonden bei der Auswertung nicht erfasst und beeinträchtigen die Messung nicht.

In einer Variante werden die Viruspartikel-Fängermolekül Komplexe zusätzlich zur Immobilisierung auf dem Substrat chemisch fixiert, d.h. Viruspartikel und Fängermolekül werden zusätzlich zur Verknüpfung über die Bindungsstelle durch chemische Bindungen, bevorzugt kovalente Bindungen miteinander verbunden, so dass eine Dissoziation verhindert wird.

In einer Alternative werden Sonden-Viruspartikel-Fängermolekül Komplexe zusätzlich zur Immobilisierung auf dem Substrat chemisch fixiert, d.h. Sonde(n), Viruspartikel und Fängermolekül werden zusätzlich zur Verknüpfung über die Bindungsstelle durch chemische Bindungen, bevorzugt kovalente Bindungen miteinander verbunden, so dass eine Dissoziation verhindert wird.

In einer anderen Alternative werden Sonden-Viruspartikel-Komplexe chemisch fixiert, d.h. Viruspartikel und Sonde(n) werden zusätzlich zur Verknüpfung über die Bindungsstelle durch chemische Bindungen, bevorzugt kovalente Bindungen miteinander verbunden, so dass eine Dissoziation verhindert wird. Anschließend erfolgt die Immobilisierung auf dem Substrat.

In einer Ausführung sind die Bindungsstellen der Viruspartikel Epitope und die Fängermoleküle und/oder Sonden Antikörper oder Aptamere oder Kombinationen davon. In einer Variante sind Fängermoleküle und/oder Sonden Antikörper. In einer Variante der vorliegenden Erfindung können Fängermoleküle und Sonden identisch sein.

In einer Ausführung der vorliegenden Erfindung unterscheiden sich Fängermoleküle und Sonden. So können z.B. unterschiedliche Antikörper als Fängermoleküle und Sonden eingesetzt werden. In einer weiteren Ausführung der vorliegenden Erfindung werden Fängermoleküle und Sonden eingesetzt, die mit Ausnahme der eventuellen (Farbstoff-) Markierung identisch zueinander sind. In einer Alternative der vorliegenden Erfindung werden verschiedene Sonden eingesetzt, die mit Ausnahme der eventuellen (Farbstoff-) Markierung identisch zueinander sind. In weiteren Alternativen der vorliegenden Erfindung werden mindestens zwei oder mehrere unterschiedliche Fängermoleküle und/oder Sonden eingesetzt, die unterschiedlichen Antikörper enthalten und gegebenenfalls auch unterschiedliche Farbstoffmarkierung haben.

Zur Detektion sind die Sonden so gekennzeichnet, dass sie ein optisch detektierbares Signal aussenden, ausgewählt aus der Gruppe bestehend aus Fluoreszenz-, Biolumineszenz- und Chemolumineszenz-Emission sowie Absorption.

In einer Alternative sind die Sonden somit mit Fluoreszenzfarbstoffen markiert. Als Fluoreszenzfarbstoff können die dem Fachmann bekannten Farbstoffe eingesetzt werden. Alternativ können auch fluoreszente Biomoleküle, bevorzugt GFP (Green Fluorescence Protein), Konjugate und/oder Fusionsproteine davon, sowie Quantendots verwendet werden. Zur späteren Qualitätskontrolle der Oberfläche, zum Beispiel Gleichmäßigkeit der Beschichtung mit Fängermolekülen, können Fängermoleküle, gekennzeichnet mit Fluoreszenzfarbstoffen, eingesetzt werden. Hierzu wird bevorzugt ein Farbstoff verwendet, der nicht mit der Detektion interferiert. Dadurch wird eine nachträgliche Kontrolle des Aufbaus möglich sowie eine Normierung der Messergebnisse.

Die Detektion der immobilisierten und markierten virusähnlichen oder Viruspartikel erfolgt mittels Abbildung der Oberfläche (z.B.: Laser Mikroskopie). Eine möglichst hohe Ortsauflösung ermittelt eine hohe Anzahl an Bildpunkten, wodurch die Sensitivität sowie die Selektivität des Assays erhöht werden können, da strukturelle Merkmale mit abgebildet und analysiert werden können. Somit erhöht sich das spezifische Signal vor dem Hintergrundsignal (z.B. unspezifisch gebundener Sonden).

Die Detektion erfolgt bevorzugt mit konfokaler Fluoreszenzmikroskopie, Fluoreszenzkorrelationsspektroskopie (FCS), insbesondere in Kombination mit Kreuzkorrelation und Laser-Scanning-Mikroskop (LSM).

Die Detektion wird in einer Alternative der vorliegenden Erfindung mit einem konfokalen Laser-Scanning-Mikroskop durchgeführt.

In einer Ausführung der vorliegenden Erfindung wird ein Laserfokus, wie er z.B. in der Laser-Scanning-Mikroskopie (LSM) verwendet wird, oder ein FCS (Fluorescence Correlation Spectroscopy System), dazu eingesetzt, sowie die entsprechenden superauflösenden Varianten wie zum Beispiel STED, PALM oder SIM.

In einer weiteren Ausführung kann die Detektion mittels ortsauflösender Fluoreszenzmikroskopie, bevorzugt durch ein TIRF-Mikroskop erfolgen, sowie die entsprechenden superauflösenden Varianten davon, wie zum Beispiel STORM, dSTORM. Mithin wird zur Detektion bevorzugt LSM und/oder TIRF eingesetzt, besonders bevorzugt TIRF.

Im Unterschied zu ELISA entstehen durch diese Verfahren so viele Readout-Werte, wie Orts-aufgelöste Ereignisse (z.B. Pixel) vorhanden sind. Abhängig von der Anzahl an unterschiedlichen Sonden wird diese Information sogar vervielfacht. Diese Vervielfachung gilt für jedes Detektionsereignis und führt zu einem Informationsgewinn, da sie weitere Eigenschaften (z.B. zweites Merkmal) über virusähnliche oder Viruspartikel offenbart. Durch so einen Aufbau kann für jedes Ereignis die Spezifität des Signales erhöht werden.

Die Sonden können derart ausgewählt werden, dass die Anwesenheit von einzelnen Virus-Bestandteilen, (z.B. einzelne Hüllen/Capsid-Proteinmoleküle), das Messergebnis nicht beeinflussen. Die Sonden können derart ausgewählt werden, dass Virus-Spezies und -Subspezies (Sero-Typen) für jedes einzelne Viruspartikel bestimmt werden können. Zusätzliche Senden können derart ausgewählt werden, dass die Unterscheidung zwischen DNA/ RNA-enthaltenden und "leeren' Virus-Hüllen möglich wird, z.B. durch DNA/RNAbindende Fluorophore (EtBr, Etl).

Zur Auswertung werden die ortsaufgelösten Informationen (z.B. die Fluoreszenz-Intensität) aller eingesetzten und delektierten Sonden herangezogen, um z.B. die Anzahl virusähnlichen oder Viruspartikel, deren Größe und deren Merkmale zu bestimmen. Dabei können z.B. auch Algorithmen der Hintergrundminimierung und/oder auch Intensitäts-Schwellenwerte für die weitere Auswertung sowie der Mustererkennung angewandt werden. Weitere Bildanalyse-Optionen beinhalten z.B. die Suche nach lokalen Intensitätsmaxima, um aus der Bildinformation die Zahl an detektierten Viruspartikeln zu erhalten.

Um die Testergebnisse miteinander (über Entfernungen, Zeiten und Experimentatoren hinweg) vergleichbar zu machen, können Standards (interne und/oder externe) eingesetzt werden.

In einer Ausführung der Erfindung werden somit Standards verwendet, insbesondere Standards, wie sie in der WO 2016/146093 A beschrieben werden. Diese werden bevorzugt als Kalibrationsstandards und/oder interne Standards eingesetzt. Erfindungsgemäß werden hierzu Standards eingesetzt, die der Größe der Viren entsprechen, mithin zwischen 10 und 500 nm im Durchmesser, bevorzugt 20 - 200 nm Durchmesser. Bevorzugt wird als Grundkörper ein anorganisches Nanopartikel verwendet, an welches in einem Verfahren gemäß der o.g. WO 2016/146093 A Teile der Hüllenproteine von Viruspartikel die Epitope enthalten oder daraus bestehen, gebunden werden.

In einer Ausführung wird ein solcher Standards in folgenden Schritten hergestellt:
A) Bereitstellen eines anorganischen Nanopartikels mit der Größe des zu analysierenden Viruspartikels (10-500 nm, bevorzugt 20-200 nm),
B) Bildung freier Aminogruppen oder freier Carboxygruppen an der Oberfläche des Nanopartikels, zur Funktionalisierung der Nanopartikel-Oberfläche zu einem amin- oder carboxyfunktionalisiertem Nanopartikel,
C)
   i) Bindung von Maleinimido-Spacer-Carbonsäure an die freien Aminogruppen in Schritt B), oder
   ii) Überführung der freien Carboxygruppen in Schritt B) in NHS-Ester,
D) Bindung von Hüllen oder Teilen von Hüllen enthaltend oder bestehend aus mindestens einem Epitop
   i) an die Maleinimido-Spacer-Carbonsäuren über eine Sulfhydrylgruppe am freien Ende der Hüllen oder Teilen von Hüllen enthaltend oder bestehend aus mindestens einem Epitop , oder
   ii) an die NHS-Ester über die Aminogruppe am freien Ende der Hüllen oder Teilen von Hüllen enthaltend oder bestehend aus mindestens einem Epitop.

Verfahren wie oben beschrieben, gekennzeichnet durch Stöbersynthese in Schritt A) zur Herstellung eines Silica-Nanopartikels.

Verfahren wie oben beschrieben, gekennzeichnet durch Silanisierung der Oberfläche mit Aminopropyltriethoxysilan in Ethanol zur Bildung freier Aminogruppen in Schritt B). Verfahren nach einem der oben beschriebenen Verfahren, gekennzeichnet durch Reaktion der Aminogruppen mit Bernsteinsäureanhydrid zur Bildung freier Carboxygruppen in Schritt B).

Verfahren nach einem der oben beschriebenen Verfahren, bei der die Maleinimido-Spacer-Carbonsäure in einen NHS-Ester überführt wird bevor diese in Schritt C) i) an die freie Aminogruppe von Schritt B) gebunden wird.

Verfahren nach einem der oben beschriebenen Verfahren, gekennzeichnet durch eine Reaktion der Maleinimido-Spacer-Carbonsäure aus Schritt C) i) oder der freien Carboxygruppen aus Schritt B) mit 1-Ethyl-3-(3-dimethylaminopropyl)carbodiimid und/oder N-Hydroxysuccinimid zur Überführung in einen NHS-Ester vor dem Schritt C) i) oder in Schritt C) ii).

Verfahren nach einem der oben beschriebenen Verfahren, gekennzeichnet durch kovalente Bindung der Aminogruppe der Hüllen oder Teilen von Hüllen enthaltend oder bestehend aus mindestens einem Epitop mit dem NHS-Ester in Schritt D ii).

Verfahren nach einem der oben beschriebenen Verfahren, dadurch gekennzeichnet, dass nach der Durchführung von Schritt C) ii) ein Streptavidin-Molekül an den NHS-Ester des Nanopartikels und vor Schritt D) eine Biotingruppe an die Hüllen oder Teilen von Hüllen enthaltend oder bestehend aus mindestens einem Epitop des Proteinaggregats angeordnet wird.

In einer Ausführung der vorliegenden Erfindung kann mit dem erfindungsgemäßen Verfahren die Anzahl der Viruspartikel in einer Suspension ohne Verwendung von Kalibrationsstandards oder externen beziehungsweise internen Standards erfolgen. Hierzu wird optional zunächst eine Konzentrationsreihe von seriell verdünnten Viruspartikeln hergestellt. Diese wird mit dem erfindungsgemäßen Verfahren analysiert. Dabei werden pro RK an unterschiedlichen Positionen (zum Beispiel an 5 x 5 Positionen) je zwei Bilder in zwei Fluoreszenzkanälen (Ex/Em=635/705 nm und 488/525 nm) konsekutiv aufgenommen. Für beide Kanäle kann die maximale Laserleistung (100%), Belichtungszeit von 500 ms und ein Verstärkungswert von 800 gewählt werden. Die Bilddaten werden danach ausgewertet. Dafür werden die Intensitätsschwellenwerte für jeden Kanal anhand der negativ Kontrolle ermittelt. Für diesen Schwellenwert werden sämtliche Bilder der negativ Kontrolle je Kanal gemittelt und jener Intensitätswert ermittelt über dem sich nur noch 0,1% der gesamten Pixel (ergo 1000 Pixel) befinden. Im Auswertungsschritt wird zunächst für jedes Bild in jedem Kanal der Intensitätsschwellenwert angewendet und anschließen Bilder gleicher Position in beiden Werten miteinander verglichen. Es werden nur jene Pixel pro Bild gezählt bei denen in beiden Kanälen die Pixel an der exakt gleichen Position über dem Intensitätsschwellenwert des Kanals liegt. Abschließend wird die Anzahl der Pixel über alle Bilder in jedem RK gemittelt und danach die Mittelwerte der Mittleren Pixelzahlen der Replikatwerte ermittelt und die Standardabweichung angegeben.

Bei einer weiteren Auswertung werden die Graustufenwerte jedes korrespondierenden Pixels aus beiden Bildern je Aufnahme multipliziert, beiden Fluoreszenzfarbkanälen gewonnen wurden. Das so entstandene Bild, bzw. die Graustufenwertematrix wird anhand einer 2D-Gaussfunktion ("imgaussfilt") bei einer Standardabweichung von wenigen, zum Beispiel 4 Pixeln geglättet. Anschließend erfolgt die Bestimmung der lokalen Maxima anhand der Funktion "imregionalmax". Die Anzahl der lokalen Maxima über einem Schwellenwert entspricht hierbei der Anzahl der Partikel, wobei der Schwellenwert dem oben ermittelten entspricht. Auf diese Weise wird für jede Aufnahme die Anzahl der Partikel bestimmt und der Mittelwert über RK gleicher Konzentration gebildet. Damit ist es möglich die absolute Anzahl der Viruspartikel anzugeben. Eine genaue Beschreibung ist den Beispielen 5 und 6 zu entnehmen.

Gegenstand der vorliegenden Erfindung ist auch Verfahren zur Bestimmung der absoluten Anzahl von Viruspartikel in einer Suspension ohne Verwendung von Standard, isbesondere ohne Verwendung von Kalibrationsstandards oder externen beziehungsweise internen Standards.

Gegenstand der vorliegenden Erfindung ist auch die Verwendung eines Kits gemäß Anspruch 15 enthaltend eine oder mehrere der folgenden Komponenten:
Substrat, ggf. mit hydrophiler Oberfläche, Fängermolekül, Sonde, Substrat mit Fängermolekül, Lösungen, Puffer.

Die Verbindungen und/oder Komponenten des Kits der vorliegenden Erfindung können in Behältern gegebenenfalls mit/in Puffern und/oder Lösung, verpackt sein. Alternativ können einige Komponenten in demselben Behälter verpackt sein. Zusätzlich dazu oder alternativ dazu könnten eine oder mehrere der Komponenten an einem festen Träger, wie z.B. einer Glasplatte, einem Chip oder einer Nylonmembran oder an die Vertiefung einer Mikrotitterplatte, absorbiert sein. Ferner kann das Kit Anweisungen für den Gebrauch des Kits für eine Beliebige der Ausführungsformen enthalten.

In einer weiteren Variante des Kits sind auf dem Substrat die oben beschriebenen Fängermoleküle immobilisiert. Zusätzlich kann das KIT Lösungen und/oder Puffer enthalten. Zum Schutz der Biomolekül-abstoßenden Oberfläche (z.B. Dextranoberfläche) und/oder der darauf immobilisierten Fängermoleküle können diese mit einer Lösung oder einem Puffer überschichtet werden. In einer Alternative enthält die Lösung ein oder mehrere Biozide, welche die Haltbarkeit der Oberfläche erhöhen.

Weiterer Gegenstand der vorliegenden Offenbarung ist die Verwendung des erfindungsgemäßen Verfahrens zum Nachweis von Virus- und Virus-ähnlichen Partikeln in beliebigen Proben, zur Quantifizierung (Titerbestimmung) von Virus- und Virus-ähnlichen Partikeln, Nachweis einer viralen Infektion, Nachweis einer viralen Kontamination, Einsatz in der antiviralen Wirkstoffentwicklung, direkte und absolute Quantifizierung der Partikelzahl, Therapie-begleitende Diagnostik (target engagement), Analyse der Virus-Assemblierung, Differentialdiagnostik, Nachweis von Protein-Protein-Interaktion (Wirt/Virus) und/oder Typisierung von Viren.

Weiterer Gegenstand der vorliegenden Offenbarung ist die Verwendung des erfindungsgemäßen Verfahrens zur Überwachung von Therapien der infektiösen Erkrankungen sowie zur Überwachung und/oder Überprüfung der Wirksamkeit von Wirkstoffen und/oder Heilverfahren, z.B. über die Titerbestimmung der Virus- oder Virusähnlichen Partikel. Dies kann bei klinischen Tests, Studien als auch beim Therapie-Monitoring eingesetzt werden. Hierzu werden Proben gemäß dem erfindungsgemäßen Verfahren vermessen und die Ergebnisse verglichen.

Weiterer Gegenstand der vorliegenden Offenbarung ist die Verwendung des erfindungsgemäßen Verfahrens zur Bestimmung der Wirksamkeit von Wirkstoffen gegen Viren bei dem die Ergebnisse von Proben miteinander verglichen werden. Bei den Proben handelt es sich um Körperflüssigkeiten, entnommen vor beziehungsweise nach, oder zu unterschiedlichen Zeitpunkten nach Gabe der Wirkstoffe beziehungsweise Durchführung des Heilverfahrens. Erfindungsgemäß werden die Ergebnisse mit einer Kontrolle verglichen, die nicht dem Wirkstoff und/oder Heilverfahren unterworfen wurde. Anhand der Ergebnisse werden Wirkstoffe und/oder dessen Dosis und/oder Heilverfahren ausgewählt.

Weiterer Gegenstand der vorliegenden Offenbarung ist die Verwendung des erfindungsgemäßen Verfahrens zur Bestimmung ob eine Person in klinische Studie aufgenommen wird. Hierzu werden Proben gemäß dem erfindungsgemäßen Verfahren vermessen und in Bezug auf einen Grenzwert die Entscheidung getroffen.

### Beispiele:

### Beispiel 1

Das Experiment wurde in kommerziell erhältlichen 3-D NHS Mikrotiter Platten (PolyAn GmbH) mit 384 Reaktionskammern (RK) durchgeführt. Die RK der Mikrotiterplatten wurden mit Antikörpern: Klon anti gp8-E1 (Prod# ABIN793840, Lot# 77410, antibodies-online.com) als Fängermolekül beschichtet (15µl; 10 µg/ml in 100 mM MES pH=4,7; Inkubation über Nacht). Danach wurde die RK einem Waschprogramm bestehend aus dreimal jeweils mit Saliner Phosphat Puffer (PBS) mit 0,1 % Tween-20 und PBS waschen und absaugen; unteruzogen. Im nächsten Schritt wurden die RK mit 50 µl Smartblock (Candor Bioscience GmbH) für 1 h bei Raumtemperatur (RT) beschichtet und nach Ablauf der Zeit wieder dem oben beschriebenen Waschprogramm unterzogen. Danach wurden jeweils 15 µl der Probe in sequentieller Verdünnung in humanem EDTA Blutplasma in vierfacher Ausführung in RK aufgetragen und bei RT inkubiert. Nach der Inkubation über Nacht wurden die RK mit dem Waschprogramm gewaschen, die RK trocken gesaugt und Detektionsantikörper aufgetragen. Die Detektionsantikörper wurden jeweils mit einer Art Fluoreszenzfarbstoff markiert. Antikörper RL-ph1 (Prod# LS-C146750, LifeSpan BioScience) wurde mit Fluoreszenzfarbstoff CF488 und Antikörper LRL-ph2 (Prod# LS-C146751, Lot# 76955, LifeSpan BioScience) wurde mit Fluoreszenzfarbstoff CF633 markiert. Die Detektionsantikörper wurden zusammen in PBS zu einer End-Konzentration von 1,25 ng/ml für jeden Antikörper verdünnt. Pro RK wurden 15 µl Antikörperlösung aufgetragen und für 1 h bei Raumtemperatur inkubiert. Nach Ablauf der Zeit wurden die Platte 5 mal mit PBS mit 0,1 % Tween-20 und 5 mal mit PBS gewaschen. Nach dem Leersaugen wurden die RK mit 20 µl Wasser aufgefüllt und die Platte mit einer Folie versiegelt.

Die Messung erfolgte im TIRF Mikroskop (Leica) mit einem 100fach Öl Immersionsobjektiv. Hierfür wurde der Glasboden der Mikrotiterplatte reichlich mit Immersions-Öl bestrichen und die Platte in eine automatisierte Bühne des Mikroskops eingebracht. Danach wurde pro RK an 5 x 5 Positionen je zwei Bilder in zwei Fluoreszenzkanälen (Ex/Em=635/705 nm und 488/525 nm) konsekutiv aufgenommen. Für beide Kanäle wurde die maximale Laserleistung (100%), Belichtungszeit von 500 ms und ein Verstärkungswert von 1300 gewählt. Die Bilddaten wurden danach ausgewertet.

Für die Analyse wurden zunächst ein Quadratischer ROI von 800 verwendet. Das bedeutet, dass jeweils die randständigsten 100 Pixel auf jeder Seite jedes Bildes nicht mit ausgewertet wurden somit aus einem 1000x1000 Pixel Bild ein 800x800 Pixel Bild entsteht. Im nächsten Schritt wurden Intensitätsschwellenwerte für jeden Kanal anhand der negativ Kontrolle ermittelt. Für diesen Schwellenwert wurden sämtliche Bilder der negativ Kontrolle je Kanal gemittelt und jener Intensitätswert ermittelt über dem sich nur noch 0,1% der gesamten Pixel (ergo 640 Pixel) befinden. Im Auswertungsschritt wurde zunächst für jedes Bild in jedem Kanal der Intensitätsschwellenwert angewandt und anschließen Bilder gleicher Position in beiden werten miteinander verglichen. Es wurden nur jene Pixel pro Bild gezählt bei denen in beiden Kanälen der Pixel an der exakt gleichen Position über dem Intensitätsschwellenwert des Kanals liegt. Abschließend wurde die Anzahl der Pixel über alle Bilder in jedem RK gemittelt und danach die die Mittelwerte der Mittleren Pixelzahlen der Replikatwerte ermittelt und die Standardabweichung angegeben.

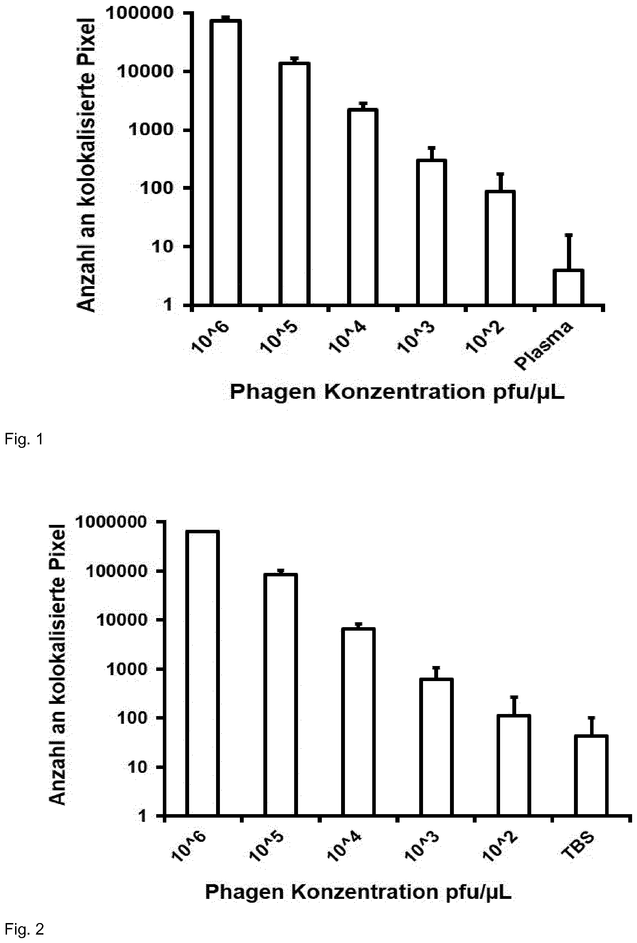

Die Ergebnisse sind in Fig. 1 zusammengefasst.

Fig. 1 zeigt eine Konzentrationsreihe an seriell verdünnten M13 Phagen (Ph.D.-7 Phage Display Library; Prod# E8102L, Lot# 0061005, New England BioLabs) in negativen humanem EDTA Blutplasma. Die Abbildung zeigt einen linearen Zusammenhang zwischen dem Messsignal und der Konzentration an M13 Phagen über 5 log Verdünnungsstufen und eine Unterscheidbarkeit der niedrigsten Konzentration zum Hintergrund.

### Beispiel 2:

Das Experiment, dessen Ergebniss in Abbildung 2 zusammengefasst ist, erfolgte analog zu Beispiel 1 mit dem Unterschied, dass die Phagen in salinen TRIS Puffer verdünnt wurden anstatt in humanem Blutplasma. Daraus ergaben sich auch unterschiedliche Intensitätsschwellenwerte, die allerdings mit der gleichen Regel (0,1 % der negative Kontrolle) ermittelt wurde. Die Auswertung erfolgte analog zu Beispiel 1.

### Beispiel 3:

zeigt die Phagen nach der Messung mit Hoechst Stain.

Für das Experiment wurden kommerzielle Mikrotiter Platten (Greiner Bio-one; Sensoplate Plus) mit 384 Reaktionskammern (RK) verwendet. Zunächst wurde die Oberfläche der Mikrotiterplatte aufgebaut. Hierfür wurde die Platte in einen Exsikator platziert in dem sich eine Schale mit 5%igem APTES in Toluol befand. Der Exsikator wurde mit Argon geflutet und eine Stunde inkubiert. Danach wurde die Schale entfernt und die Platte für 2 Stunden im Vakuum getrocknet. In die Reaktionskammern der trockenen Platte wurden 20 µl einer 2 mM Lösung an SC-PEG-CM (MW 3400; Laysan Bio) in de-ionisiertem H₂O eingefüllt und für 4 Stunden inkubiert. Nach der Inkubation wurde die RK drei mal mit Wasser gewaschen und im Anschluss mit je 20 µl einer wässrigen 200 mM EDC-Lösung (1-Ethyl-3-(3-dimethylaminopropyl)carbodiimide; Sigma) und mit 50 mM NHS (N-Hydroxysuccinimide, Sigma) für 30 Minuten inkubiert. Die Platte wurde wieder mit dreimal mit deionisertem Waser gewaschen. Danach wurden die RK mit Klon anti gp8-E1 (Prod# ABIN793840, Lot# 77410, antibodies-online.com) Antikörpern als Fängermolekül beschichtet (20µl; 10 µg/ml in PBS; 1 Stunde). Anschließend wurde die RK mit dem Waschprogramm bestehend aus jeweils dreimal Waschen und Leersaugen mit TBS mit 0,1 % Tween-20 und TBS behandelt. Im nächsten Schritt wurden die RK mit 50 µl Smartblock (Candor Bioscience GmbH) über Nacht bei Raumtemperatur (RT) beschichtet und nach Ablauf der Zeit wieder dreimal mit salinem tris(hydroxymethyl)aminomethan (TBS; pH=7,4) gewaschen und leergesaugt. Die Proben wurden sequentiell in tris(hydroxymethyl)aminomethan (TRIS) Puffer mit Farbstoff Hoechst Stain (1 µg ml-1) verdünnt und für eine Stunde inkubiert. Danach wurde in dreifacher Ausführung je 15 µl Probe in RK aufgetragen und bei RT für 1 Stunde inkubiert. Nach der Inkubation wurden die RK dreimal mit TBS gewaschen und Nach Ablauf der Zeit wurden die Platte 3 mal mit TBS gewaschen. Nach dem Leersaugen wurden die RK mit 20 µl TBS aufgefüllt und die Platte einer Folie versiegelt.

Die Messung erfolgte im TIRF Mikroskop (Leica) mit einem 100fach Öl Immersionsobjektiv. Hierfür wurde der Glasboden der Mikrotiterplatte reichlich mit Immersions-Öl bestrichen und die Platte in die automatisierte Bühne des Mikroskops eingebracht. Danach wurde pro RK an 5 x 5 Positionen ein Bild im Fluoreszenzkanal (Ex/Em=405/450 nm) konsekutiv aufgenommen. Es wurde die maximale Laserleistung (100%), eine Belichtungszeit von 500 ms und ein Verstärkungswert von 800 gewählt. Die Bilddaten wurden danach ausgewertet. Ein Intensitätsschwellenwert wurde für den Kanal bei 4000 Graustufen gesetzt. Im Auswertungsschritt wurde zunächst für jedes Bild in jedem Kanal der Intensitätsschwellenwert angewandt und anschließen Bilder gleicher Position in beiden werten miteinander verglichen. Es wurden nur jene Pixel pro Bild gezählt bei denen in beiden Kanälen der Pixel an der exakt gleichen Position über dem Intensitätsschwellenwert des Kanals liegt. Abschließend wird die Anzahl der Pixel über alle Bilder in jedem RK gemittelt und danach die Mittelwerte der Mittleren Pixelzahlen der Replikatwerte ermittelt und die Standardabweichung angegeben.

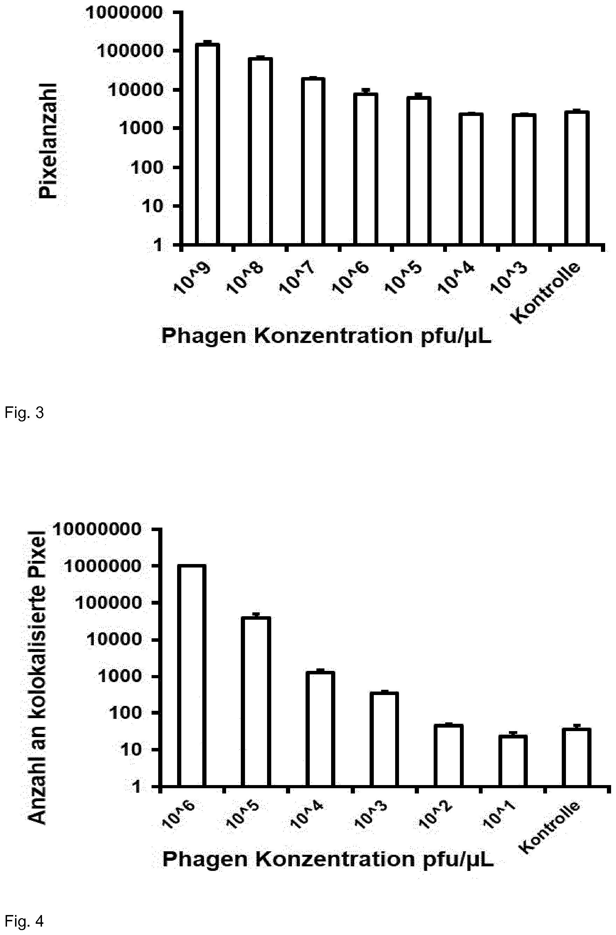

Die Ergebnisse sind in Fig. 3 zusammengefasst.

Fig. 3 zeigt eine Konzentrationsreihe an seriell verdünnten M13 Phagen (Ph.D.-7 Phage Display Library; Prod# E8102L, Lot# 0061005, New England BioLabs) in TBS mit Hoechst Stain. Die Abbildung zeigt Konzentrationsabhängige Zusammenhang an M13 Phagen über 5 log Verdünnungsstufen (10^9 - 10^5) und die letzten beiden Konzentrationsstufen ließen sich nicht mehr vom Hintergrund unterscheiden.

Es zeigt eindeutig die Eignung von Hoechst Stain als Farbstoff der an DNA und RNA im Virus Assay haftet um die Viruspartikel anzufärben. Somit könne intakte Viren nachgewiesen werden, beziehungsweise zwischen leeren Hüllen und Hüllen enthaltend DNA und/oder RNA unterschieden werden.

### Beispiel 4:

Fig. 4 zeigt die Phagen aus Abbildung 3 nach der Messung mit Höchst Stain. Die RK der Mikroplatte aus Beispiel 3 wurden dreimal mit TBS gewaschen und mit den Detektionsantikörper 1,25 µg/ ml RL-ph1 (Prod# LS-C146750, LifeSpan BioScience) markiert mit dem CF488 Fluoreszenzfarbstoff und 1,25 µg/ ml LRL-ph2 (Prod# LS-C146751, Lot# 76955, LifeSpan BioScience) markiert mit CF633 Fluoreszenzfarbstoff für 1 Stunde inkubiert. Nach dreimaligem Waschen mit TBS wurden die Proben TIRF Mikroskop (Leica) mit einem 100fach Öl Immersionsobjektiv gemessen. Hierfür wurde der Glasboden der Mikrotiterplatte reichlich mit Immersions-Öl bestrichen und die Platte in die automatisierte Bühne des Mikroskops eingebracht. Danach wurde pro RK an 5 x 5 Positionen je zwei Bilder in zwei Fluoreszenzkanälen (Ex/Em=635/705 nm und 488/525 nm) konsekutiv aufgenommen. Für beide Kanäle wurde die maximale Laserleistung (100%), Belichtungszeit von 500 ms und ein Verstärkungswert von 800 gewählt. Die Bilddaten wurden danach ausgewertet. Dafür wurden Intensitätsschwellenwerte für jeden Kanal anhand der negativ Kontrolle ermittelt. Für diesen Schwellenwert wurden sämtliche Bilder der negativ Kontrolle je Kanal gemittelt und jener Intensitätswert ermittelt über dem sich nur noch 0,1% der gesamten Pixel (ergo 1000 Pixel) befinden. Im Auswertungsschritt wurde zunächst für jedes Bild in jedem Kanal der Intensitätsschwellenwert angewandt und anschließen Bilder gleicher Position in beiden Werten miteinander verglichen. Es wurden nur jene Pixel pro Bild gezählt bei denen in beiden Kanälen die Pixel an der exakt gleichen Position über dem Intensitätsschwellenwert des Kanals lag. Abschließend wurde die Anzahl der Pixel über alle Bilder in jedem RK gemittelt und danach die Mittelwerte der Mittleren Pixelzahlen der Replikatwerte ermittelt und die Standardabweichung angegeben.

Das Experiment zeigt sowohl den konzentrationsabhängige Zusammenhang an M13 Phagen über 4 log Verdünnungsstufen (10^6 - 10^3). Es zeigt, dass eine nachträgliche Anfärbung mit fluoreszenzmarkierten Antikörpern möglich ist. Ferner zeigt das Experiment, dass durch Mikrotiter Platten mit PEG Beschichtung annähernd die gleiche Sensitivität wie bei 3D NHS Platten bei einer kürzeren Inkubationszeit erreicht wird (vgl. Fig. 2).

### Beispiel 5:

Das Experiment wurde in kommerziell erhältlichen 3-D NHS Mikrotiter Platten (PolyAn GmbH) mit 384 Reaktionskammern (RK) durchgeführt. Die RK der Mikrotiterplatten wurden mit Anti VSV-G Antikörper P5D4 (Sigma) als Fängermolekül beschichtet (15µl; 10 µg/ml in 100 mM MES pH=4,7; über Nacht). Danach wurde die RK mit dem Waschprogramm bestehend aus jeweils dreimal Waschen und Leersaugen mit salinem Phosphat Puffer (PBS) mit 0,1 % Tween-20 und PBS behandelt. Im nächsten Schritt wurden die RK mit 50 µl Smartblock (Candor Bioscience GmbH) für 1 h bei Raumtemperatur (RT) beschichtet und nach Ablauf der Zeit wieder dreimal mit salinem tris(hydroxymethyl)aminomethan (TBS; pH=7,4) gewaschen und leergesaugt. Die Proben wurden sequentiell in salinem tris(hydroxymethyl)aminomethan (TBS) Puffer für eine Stunde inkubiert. Danach wurden in dreifacher Ausführung je 15 µl Probe in RK aufgetragen und bei RT für 1 Stunde inkubiert. Nach der Inkubation wurden die RK dreimal mit TBS gewaschen und leergesaugt und mit 15 µl Detektionsantikörper versetzt. Die Detektionsantikörper wurden jeweils mit einer Art Fluoreszenzfarbstoff markiert. Anti VSV-G Antikörper P5D4 (Sigma) wurden jeweils mit CF488 und mit CF633 markiert. Die Detektionsantikörper wurden zusammen in TBS zu einer finale Konzentration von 1,25 ng/ml für jeden Antikörper verdünnt. Pro RK wurden 15 µl Antikörperlösung aufgetragen und für 1 h bei RT inkubiert. Nach Ablauf der Zeit wurden die Platte 3 mal mit TBS gewaschen. Nach dem Leersaugen wurden die RK mit 20 µl TBS aufgefüllt und die Platte einer Folie versiegelt.

Die Messung erfolgte im TIRF Mikroskop (Leica) mit einem 100fach Öl Immersionsobjektiv. Hierfür wurde der Glasboden der Mikrotiterplatte reichlich mit Immersions-Öl bestrichen und die Platte in die automatisierte Bühne des Mikroskops eingebracht. Danach wurde pro RK an 5 x 5 Positionen je zwei Bilder in zwei Fluoreszenzkanälen (Ex/Em=635/705 nm und 488/525 nm) konsekutiv aufgenommen. Für beide Kanäle wurde die maximale Laserleistung (100%), Belichtungszeit von 500 ms und ein Verstärkungswert von 800 gewählt. Die Bilddaten wurden danach ausgewertet. Dafür wurden Intensitätsschwellenwerte für jeden Kanal anhand der negativ Kontrolle ermittelt. Für diesen Schwellenwert wurden sämtliche Bilder der negativ Kontrolle je Kanal gemittelt und jener Intensitätswert ermittelt über dem sich nur noch 0,1% der gesamten Pixel (ergo 1000 Pixel) befinden. Im Auswertungsschritt wurde zunächst für jedes Bild in jedem Kanal der Intensitätsschwellenwert angewandt und anschließen Bilder gleicher Position in beiden Werten miteinander verglichen. Es wurden nur jene Pixel pro Bild gezählt bei denen in beiden Kanälen die Pixel an der exakt gleichen Position über dem Intensitätsschwellenwert des Kanals lag. Abschließend wurde die Anzahl der Pixel über alle Bilder in jedem RK gemittelt und danach die Mittelwerte der Mittleren Pixelzahlen der Replikatwerte ermittelt und die Standardabweichung angegeben.

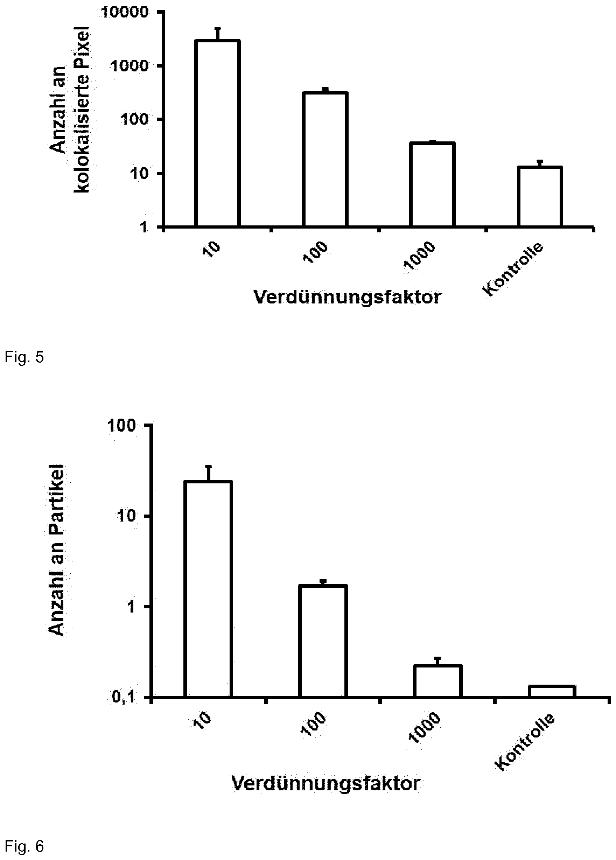

Die Ergebnisse sind in Fig. 5 zusammengefasst.

Fig. 5 zeigt eine Konzentrationsreihe an seriell verdünnten Viruspartikeln eines HIV Sicherheitsstamms (Biosynthese siehe J Mol Biol. 2017 Apr 21;429(8):1171-1191. doi: 10.1016/j.jmb.2017.03.015., der ein VSV-Glykoprotein in der Lipidmembran enthält) aus Zellkulturmedium in TBS verdünnt. Die Abbildung zeigt einen linearen Zusammenhang zwischen dem Messsignal und der Konzentration an Viruspartikel über 3 log Verdünnungsstufen und eine Unterscheidbarkeit der niedrigsten Konzentration zum Hintergrund. Da es sich hierbei um ein Viruspartikel mit Lipidhülle handelt, kann das erfindungsgemäße Verfahren auf für die Analyse dieser tauxonomen Gruppe eingesetzt werden.

### Beispiel 6:

Fig. 6 zeigt die Anzahl der Viruspartikel pro µm² in den einzelnenVerdünnungsstufen der Daten aus Fig. 5. Diese alternative Auswertung der Daten erlaubt es Viruspartikel absolut zu zählen und ist somit unabhängig von einem Standard. Für die Auswertung wurden die Graustufenwerte jedes korrespondierenden Pixels aus beiden Bildern je Aufnahme multipliziert, beiden Fluoreszenzfarbkanälen gewonnen wurden. Das so entstandene Bild, bzw. die Graustufenwertematrix wurde anhand einer 2D-Gaussfunktion ("imgaussfilt") bei einer Standardabweichung von 4 Pixeln geglättet. Anschließend erfolgte die Bestimmung der lokalen Maxima anhand der Funktion "imregionalmax". Die Anzahl der lokalen Maxima über einem Schwellenwert entspricht hierbei der Anzahl der Partikel, wobei der Schwellenwert dem in Abbildung 5 ermittelten entspricht. Auf diese Weise wurde für jede Aufnahme die Anzahl der Partikel bestimmt und final der Mittelwert über RK gleicher Konzentration gebildet.

## Patentansprüche

1. Verfahren zur quantitativen und/oder qualitativen Bestimmung von Viruspartikeln enthaltend mindestens eine Bindungsstelle für ein Fängermolekül und mindestens eine Bindungsstelle für eine Sonde umfassend folgende Schritte:
a) Immobilisieren von Fängermolekülen auf einem Substrat,
b) In Kontakt bringen der Viruspartikel mit den Fängermolekülen, wobei die Fängermoleküle kovalent an das Substrat gebunden sind,
c) Immobilisieren der Viruspartikel auf dem Substrat durch Bindung an Fängermoleküle,
d) In Kontakt bringen der Viruspartikel mit den Sonden und
e) Binden der Sonden an die Viruspartikel, wobei die Sonden ein Viruspartikel-affines Molekül oder Molekülteil, welches eine Bindungsstelle des Viruspartikels erkennt und daran bindet, umfassen,
wobei die Sonden in der Lage sind, ein spezifisches Signal zu emittieren und
die Schritte b) und d) gleichzeitig oder d) vor b) erfolgen können,
wobei eine ortsaufgelöste Bestimmung des Sondensignals erfolgt, wobei die Detektion mittels ortsauflösender Fluoreszenzmikroskopie erfolgt, wobei zur Detektion TIRF eingesetzt wird.

2. Verfahren nach Anspruch 1 **dadurch gekennzeichnet, dass** die Viruspartikel ausgewählt werden aus der Gruppe enthaltend oder bestehend aus Virus, Virion, Bakteriophage und Teile oder Fragmente davon.

3. Verfahren nach einem der vorangehenden Ansprüche **dadurch gekennzeichnet, dass** das Substrat aus Kunststoff, Silicium oder Siliciumdioxid, bevorzugt Glas ist.

4. Verfahren nach einem der vorangehenden Ansprüche **dadurch gekennzeichnet, dass** das Substrat vor Schritt a) eine hydrophile Oberfläche aufweist.

5. Verfahren nach Anspruch 4 **dadurch gekennzeichnet, dass** auf das Substrat vor Schritt a) eine hydrophile Schicht aufgetragen wird.

6. Verfahren nach Anspruch 5 **dadurch gekennzeichnet, dass** die hydrophile Schicht ausgewählt wird aus der Gruppe enthaltend oder bestehend aus PEG, Poly-Lysin und Dextran oder Derivate davon.

7. Verfahren nach Anspruch 5 **dadurch gekennzeichnet, dass** das Substrat vor dem Aufbringen der hydrophilen Schicht hydroxyliert und mit reaktiven Gruppen (Aminogruppen) funktionalisiert wird.

8. Verfahren nach Anspruch 7 **dadurch gekennzeichnet, dass** Funktionalisierung mit Aminogruppen durch in Kontaktbringen des Substrats mit APTES (3-Aminopropyltrietoxysilan) oder Ethanolamin erfolgt.

9. Verfahren nach Anspruch 8 **dadurch gekennzeichnet, dass** das in Kontakt bringen des Substrats mit APTES in der Gasphase erfolgt.

10. Verfahren nach einem der vorangehenden Ansprüche **dadurch gekennzeichnet, dass** zwischen leeren Virushüllen und Viruspartikeln enthaltend neben der Hülle DNA oder RNA unterschieden wird.

11. Verfahren nach einem der vorangehenden Ansprüche **dadurch gekennzeichnet, dass** die Bindungsstellen der Viruspartikel Epitope sind und die Fängermoleküle und Sonden Antikörper oder Aptamere oder Kombinationen davon sind.

12. Verfahren nach einem der vorangehenden Ansprüche **dadurch gekennzeichnet, dass** die Sonden mit Fluoreszenzfarbstoffen markiert sind.

13. Verfahren nach einem der vorangehenden Ansprüche **dadurch gekennzeichnet, dass** so viele Datenpunkte gesammelt werden, dass die Detektion eines Virus vor einem Hintergrundsignal ermöglicht wird.

14. Verfahren nach einem der vorangehenden Ansprüche **dadurch gekennzeichnet,**
**dass** die Detektion mittels konfokaler Fluoreszenzmikroskopie, Fluoreszenzkorrelationsspektroskopie (FCS), insbesondere in Kombination mit Kreuzkorrelation und Laser-Scanning-Mikroskop (LSM), erfolgt.

15. Verwendung eines Kits zur Durchführung des Verfahrens nach einem der Ansprüche 1 - 14, wobei das Kit eine oder mehrere der folgenden Komponenten enthält:
Substrat, ggf. mit hydrophiler Oberfläche, Fängermolekül, Sonde, Substrat mit Fängermolekül, Lösungen, Puffer.

## Claims

1. Method for the quantitative and/or qualitative determination of virus particles containing at least one binding site for a capture molecule and at least one binding site for a probe comprising the following steps:
a) immobilising capture molecules on a substrate,
b) contacting the virus particles with the capture molecules, wherein the capture molecules are covalently bound to the substrate,
c) immobilising the virus particles on the substrate by binding to capture molecules,
d) contacting the virus particles with the probes; and
e) binding the probes to the virus particles, wherein the probes comprise a virus particle-affine molecule or molecule part which recognises and binds to a binding site of the virus particle,
wherein said probes are capable of emitting a specific signal, and
steps b) and d) may occur simultaneously or d) may occur prior to b),
wherein a spatially resolved determination of the probe signal is carried out, wherein the detection is carried out by means of spatially resolving fluorescence microscopy, wherein TIRF is used for detection.

2. Method according to claim 1, **characterised in that** the virus particles are selected from the group comprising or consisting of virus, virion, bacteriophage and parts or fragments thereof.

3. Method according to one of the preceding claims, **characterized in that** the substrate is made of plastic, silicon or silicon dioxide, preferably glass.

4. Method according to one of the preceding claims, **characterized in that** the substrate has a hydrophilic surface prior to step a).

5. Method according to claim 4, **characterized in that** a hydrophilic layer is applied to the substrate prior to step a).

6. Method according to claim 5, **characterized in that** the hydrophilic layer is selected from the group comprising or consisting of PEG, poly-lysine and dextran or derivatives thereof.

7. Method according to claim 5, **characterized in that** the substrate is hydroxylated and functionalized with reactive groups (amino groups) prior to the application of the hydrophilic layer.

8. Method according to claim 7, **characterized in that** functionalization with amino groups is carried out by contacting the substrate with APTES (3-aminopropyltrietoxysilane) or ethanolamine.

9. Method according to claim 8, **characterized in that** the bringing into contact of the substrate with APTES takes place in the gas phase.

10. Method according to one of the preceding claims, **characterized in that** a distinction is made between empty viral envelopes and virus particles containing DNA or RNA in addition to the envelope.

11. Method according to one of the preceding claims, **characterized in that** the binding sites of the virus particles are epitopes and the capture molecules and probes are antibodies or aptamers or combinations thereof.

12. Method according to one of the preceding claims, **characterised in that** the probes are labelled with fluorescent dyes.

13. Method according to one of the preceding claims, **characterised in that** so many data points are collected that the detection of a virus in front of a background signal is made possible.

14. Method according to one of the preceding claims, **characterized in that** the detection is carried out by means of confocal fluorescence microscopy, fluorescence correlation spectroscopy (FCS), in particular in combination with cross correlation and laser scanning microscope (LSM).

15. Use of a kit for carrying out the method according to one of claims 1 - 14, wherein the kit comprises one or more of the following components:
Substrate, optionally with hydrophilic surface, capture molecule, probe, substrate with capture molecule, solutions, buffer.

## Revendications

1. Procédé de détermination quantitative et/ou qualitative de particules virales contenant au moins un site de liaison pour une molécule de capture et au moins un site de liaison pour une sonde, comprenant les étapes suivantes consistant à :
a) immobiliser des molécules de capture sur un substrat,
b) mettre en contact les particules virales avec les molécules de capture, les molécules de capture étant liées de façon covalente au substrat,
c) immobiliser les particules virales sur le substrat en les liant aux molécules de capture,
d) mettre en contact les particules virales avec les sondes ; et
e) lier les sondes aux particules virales, les sondes comprenant une molécule ou partie de molécule affine aux particules virales, qui reconnaît un site de liaison de la particule virale et qui se lie à celui-ci,
les sondes étant capables d'émettre un signal spécifique, et les étapes b) et d) pouvant être réalisées simultanément, ou d) avant b),
une détermination à résolution locale du signal de sonde étant effectuée, la détection étant effectuée à l'aide de la microscopie de fluorescence à résolution locale, la détection utilisant la microscopie TIRF.

2. Procédé selon la revendication 1,
**caractérisé en ce que** les particules virales sont choisies dans le groupe comprenant ou constitué d'un virus, d'un virion, d'un bactériophage et de parties ou fragments de ceux-ci.

3. Procédé selon l'une des revendications précédentes,
**caractérisé en ce que** le substrat est en matière plastique, en silicium ou en silice, de préférence en verre.

4. Procédé selon l'une des revendications précédentes,
**caractérisé en ce que** le substrat présente une surface hydrophile avant l'étape a).

5. Procédé selon la revendication 4,
**caractérisé en ce qu'**une couche hydrophile est appliquée sur le substrat avant l'étape a).

6. Procédé selon la revendication 5,
**caractérisé en ce que** la couche hydrophile est choisie dans le groupe comprenant ou constitué de PEG, de poly-lysine et de dextrane ou de leurs dérivés.

7. Procédé selon la revendication 5,
**caractérisé en ce que** le substrat est hydroxylé et fonctionnalisé avec des groupes réactifs (groupes amino) avant l'application de la couche hydrophile.

8. Procédé selon la revendication 7,
**caractérisé en ce que** la fonctionnalisation avec des groupes amino est effectuée par la mise en contact du substrat avec de l'APTES (3-aminopropyltriétoxysilane) ou de l'éthanolamine.

9. Procédé selon la revendication 8,
**caractérisé en ce que** la mise en contact du substrat avec l'APTES est effectuée en phase gazeuse.

10. Procédé selon l'une des revendications précédentes,
**caractérisé en ce que** l'on fait la distinction entre les enveloppes virales vides et les particules virales contenant, outre l'enveloppe, de l'ADN ou de l'ARN.

11. Procédé selon l'une des revendications précédentes,
**caractérisé en ce que** les sites de liaison des particules virales sont des épitopes, et les molécules de capture et les sondes sont des anticorps ou des aptamères ou des combinaisons de ceux-ci.

12. Procédé selon l'une des revendications précédentes,
**caractérisé en ce que** les sondes sont marquées avec des colorants fluorescents.

13. Procédé selon l'une des revendications précédentes,
**caractérisé en ce qu'**un nombre suffisant de points de données est collecté pour permettre la détection d'un virus devant un signal de fond.

14. Procédé selon l'une des revendications précédentes,
**caractérisé en ce que** la détection s'effectue par microscopie à fluorescence confocale, par spectroscopie de corrélation de fluorescence (FCS), en particulier en combinaison avec une corrélation croisée et un microscope à balayage laser (LSM).

15. Utilisation d'un kit pour mettre en oeuvre le procédé selon l'une des revendications 1 à 14, le kit comprenant un ou plusieurs des composants suivants :
substrat, le cas échéant avec une surface hydrophile, molécule de capture, sonde, substrat avec molécule de capture, solutions, tampon.
